# EUROPÄISCHE PATENTSCHRIFT

(11) **EP 3 064 924 B1**
(45) Veröffentlichungstag und Bekanntmachung des Hinweises auf die Patenterteilung: **26.04.2017**
(21) Anmeldenummer: 16000309.1
(22) Anmeldetag: 06.02.2016
(51) Int. Cl.: G01N 1/20, F16K 37/00, F16L 29/00, G01K 5/48

(54) **PROBEENTNAHMEVENTIL**
SAMPLING VALVE
SOUPAPE DE PRELEVEMENT D'ECHANTILLON

(30) Priorität: 05.03.2015 DE 102015002854
(43) Veröffentlichungstag der Anmeldung: 07.09.2016
(73) Patentinhaber: Fidica GmbH & Co. KG, 63877 Sailauf (DE)
(72) Erfinder: VÖLKER, Manfred, 63825 Blankenbach (DE)
(74) Vertreter: Flosdorff, Jürgen

(56) Entgegenhaltungen:
- EP-A1- 0 041 755
- EP-A1- 2 475 973
- DE-A1-102008 019 982
- DE-U1-202015 003 364

## Beschreibung

Ziel dieser Entwicklung ist es eine Probeentnahmevorrichtung für Flüssigkeiten zu entwickeln, die eine Probeentnahme möglichst ohne mikrobiologische und oder chemische Beeinflussung durch den Probenehmer oder die Konstruktion der Entnahmeeinheit ermöglicht.

Probeflüssigkeiten werden mittels einer Vielzahl von Probeentnahmeventilen zum Zwecke chemischer und oder mikrobiologischer und auch physikalischer oder anderer sensorischer Nachweise entnommen.

In vielen Fällen sind die regulativen und normativen Anforderungen bzgl. der mikrobiologischen und der chemischen Grenzen so hoch, dass schon kleine Verunreinigungen der Probe, z.B. vor Ort während der Probeentnahme, eine nachweissichere Verifizierung erschweren oder gar unmöglich machen.

Zum einen liegt dies an dem Mangel an Totraumfreiheit oder auch an der schlechten Reinigungsmöglichkeit der verfügbaren Konstruktionen.

Mangelnde Hygiene durch den Probenehmer wegen Kontakt mit flüssigkeitsführenden hochreinen Komponenten oder auch der Flüssigkeitsprobe selber führen zu Probeverfälschungen.

Weitere nicht zu vernachlässigende Nachteile sind nicht sterile Probebehälter, deren Identifikation und ein sicherer Transport zum Labor.

Die EP 2 475 973 A1 offenbart ein Probeentnahmeventil mit einem Ventilkörper, der in einem Ventilgehäuse zwischen einer Schließstellung und einer Öffnungsstellung des Probeentnahmeventils bewegbar ist, wobei der Ventilkörper einen Probenflüssigkeitskanal aufweist und aus einem Material mit einem hohen Wärmeleitfähigkeitskoeffizienten besteht. Außerdem offenbart die Druckschrift einen Temperatursensor, der mit dem Ventilkörper über eine Anschlussbuchse in Berührung steht und anzeigt, wann eine Sterilisierungstemperatur erreicht ist.

Die EP 0 041 755 offenbart eine Signaleinheit mit einem topfförmigen Gehäuseteil, das mit einem Elektrodenstab in Berührung steht und einen Signalknopf enthält, der von einer Schraubenfeder bei deren Erwärmung aus einem Gehäuse vorschiebbar ist.

Die DE 10 2008 019 982 A1 offenbart ein Probeentnahmeventil mit einem Ventilkörper, der in einem Ventilgehäuse zwischen einer Schließstellung und einer Öffnungsstellung des Probeentnahmeventils bewegbar ist, wobei der Ventilkörper einen Probenflüssigkeitskanal aufweist und wobei der Ventilkörper aus einem Material mit einem hohen Wärmeleitfähigkeitskoeffizienten besteht. Dieses Ventil kann durch Abflammen sterilisiert werden.

Der vorliegenden Erfindung liegt die Aufgabe zugrunde, ein Probeentnahmeventil anzugeben, dann eine sichere, anwenderfreundliche Probeentnahme ermöglicht.

Diese Aufgabe wird erfindungsgemäß durch die Merkmale des Patentanspruchs 1 gelöst.

Vorteilhafte Weiterbildungen der Erfindung sind in den Unteransprüchen gekennzeichnet.

Die Erfindung sieht vor, dass der Ventilkörper aus Metall besteht, und dass eine Signaleinheit mit einem Gehäuseteil aus Metall mit dem Ventilkörper in Berührung steht. Ein geeignetes Material ist Aluminium oder eine Aluminiumlegierung.

Außerdem sieht die Erfindung vor, dass die Signaleinheit einen Signalknopf enthält, der von einer Federeinrichtung aus einem Gehäuse, vorschiebbar ist. Das Gehäuse besteht aus dem oben erwähnten Gehäuseteil aus Metall und einem anschließenden, im wesentlichen zylindrischen Gehäuseteil, das vorzugsweise aus Kunststoff besteht und deshalb nicht oder kaum erwärmt wird.

Die Federeinrichtung ist eine Formgedächtnisfeder, die bei Erkalten wieder in ihre ursprüngliche Form zurück kehrt.

Mit großem Vorteil wird vorgeschlagen, dass außen auf dem Ventilkörper ein Griffstück aus einem wärmeisolierenden Material sitzt, das verhindert, dass sich eine Bedienungsperson bei einem Abflammvorgang des Probeentnahmeventils eine Hand verbrennt.

In weiteren Einzelheiten ist vorgesehen, dass in dem Gehäuse ein mit einem Bund versehener Führungskörper verschieblich angeordnet ist, an dessen freiem Ende der Signalknopf befestigt ist. Die Federeinrichtung, bevorzugt Formgedächtnisfedereinrichtung, stützt sich am Boden des topfförmigen Gehäuses und an dem Bund ab.

Außerdem ist in dem Gehäuse eine Rückstellfeder angeordnet, die eine kleinere Federkraft hat als die Formgedächtnisfeder und die den Führungskörper in die zurückgezogene Ausgangsposition vorspannt.

Der Signalknopf kann eine andere Farbe haben als der Führungskörper. Wenn bei einer entsprechenden Erwärmung des Ventilkörpers der Signalknopf und ein Endabschnitt des Führungskörpers über das Griffstück hinaus vorgeschoben werden, wird dies durch die andere Farbe des Führungskörpers eindeutig sichtbar.

Eine große Bedeutung kommt dabei der totraumfreien Konstruktion und deren Beurteilung bzgl. des hygienischen Zustands zu.

Eine besondere Herausforderung ist die Herstellung einer möglichst vollständigen Keimfreiheit der flüssigkeitsführenden Probenkanäle vor Beginn der Entnahme. Dabei soll es bei der Verwendung von Abflammgeräten zu keinen Schäden in der Umgebung, bzw. Verbrennungen, und auch nicht zu einem übermäßigen Einsatz von Primärenergie kommen.

Vorteilhafterweise kommt eine Entnahmetechnik zum Einsatz, die auch bei problematischen Flüssigkeiten eine sichere Flüssigkeitsführung direkt ohne Luftkontakt in den Probebehälter zulässt.

Die Lösung der Aufgabe wird vorbereitet, indem in die flüssigkeitsführende Leitung ein Ventil oder Anschlussblock mit einer Aufnahme für ein Probeentnahmeventil eingebaut wird.

Die wirkungsvolle Lösung ist, dass die zur Probe anstehende Flüssigkeit das Probeentnahmeventil an der Entnahmeseite ohne Totraum umspült.

Im geschlossenen Zustand verhindert eine konische Dichtungsfläche das Austreten von Probeflüssigkeit.
Im geöffneten Zustand fließt die Probeflüssigkeit zunächst durch den sterilisierbaren Probeflüssigkeitskanal, dann über einen aufsteckbaren sterilen Anschlussadapter und einen anzuschließenden sterilen Einmalartikel zum Probebehälter.
Mit Vorteil ist der Probeflüssigkeitskanal mit einem zentrierbaren Abflammgerät hin bis zum Entnahmeort zu sterilisieren. Dabei kann mit großem Vorteil das Sterilisationsergebnis optisch anhand eines Signalknopfes beurteilt werden.

Die Bewegung des Signalknopfes heraus aus der Ruhe- in die Sterilposition während der Erwärmung wird durch eine Feder hervorgerufen die aus einer Formgedächtnislegierung beispielsweise NiTi (Nickel-Titan, Nitinol) besteht.

Formgedächtnislegierungen sind spezielle Metalle, die in zwei unterschiedlichen Kristallstrukturen existieren können. Sie werden oft auch als Memorymetalle bezeichnet. Dies rührt von dem Phänomen her, dass sie sich an eine frühere Formgebung trotz nachfolgender starker Verformung scheinbar "erinnern" können.

Dabei gibt es Legierungen mit einem Einwegeffekt z.B. eine einmalige Formänderung beim Aufheizen. Formgedächtnislegierungen sind auch mit Zweiweg-Effekt einsetzbar. Allerdings ist dieses Bauelement beim Abkühlen nicht in der Lage, Arbeit zu verrichten.

In der erfindungsgemäßen Bauform sind beide Legierungsformen verwendbar, weil eine Rückstellfeder den Signalknopf beim Erkaltungsvorgang in die Ruheposition drückt.

Alternativ zu Formgedächtnislegierungen sind auch andere temperatursensitive Bauelemente wie z.B. Bimetalle denkbar.

Weitere Vorteile der Einheit sind der Isolationsschutz und die daraus resultierende geringe Oberflächentemperatur des Probeentnahmeventils nach dem Abflammvorgang, die damit in Zusammenhang stehende mögliche manuelle Bedienung ohne Werkzeug, die konische Dichtung hin zur flüssigkeitsführenden Leitung, die Restmengen minimierende trompetenförmige Konstruktion des Probenflüssigkeitskanals, und der universelle Anschlussadapter für den sterilen Einmalartikel.

Nach einem anderen Aspekt wird ein Anschlussadapter vorgeschlagen, der mit unterschiedlichen Ventilen verbindbar ist, auf deren Endabschnitte der Adapter aufsetzbar ist.

Der Adapter hat ein napf- oder topfförmiges Gehäuse, an dessen Boden ein Schlauchanschluss-Stutzen angesetzt ist, wobei an der Innenwand des Gehäuses eine ringförmige Dichtung anliegt, deren Innendurchmesser vom Boden des Gehäuses aus bereichsweise größer wird.

Dabei kann die ringförmige Dichtung mehrere zylindrische Abschnitte mit unterschiedlichen Durchmessern aufweisen, wobei auch wenigstens ein konisch sich erweiternder Abschnitt ausgebildet sein kann.

Damit ist der Adapter auf Ventile aufsetzbar, deren Endabschnitte unterschiedliche Durchmesser haben, so dass jeweils eine dichte Verbindung zwischen dem Adapter und dem jeweiligen Ventil herstellbar ist.

Mit besonderem Vorteil wird vorgeschlagen, dass sich der Schlauchanschluss-Stutzen des Adapters zu einem ins Innere des napfförmigen Gehäuses verlaufenden, axialen Dorn mit geschlossener Stirnwand fortsetzt. Hierdurch ist der Adapter auch zur Verbindung mit solchen Ventilen geeignet, die ein federbeaufschlagtes Verschlusselement haben, das zum Öffnen des Ventils zurück gedrückt werden muss. Dies geschieht beim Aufsetzen des Adapters durch den inneren axialen Dorn, der mit seiner geschlossenen Stirnwand gegen das Verschlusselement drückt und dieses in die Öffnungsstellung versetzt.

In der Umfangswand des Dorns des Adapters ist vorteilhafterweise wenigstens eine Öffnung zum Eintritt von Flüssigkeit ausgebildet, wobei die Öffnung mit dem Inneren des Schlauchanschluss-Stutzens in Verbindung steht, so dass die Flüssigkeit durch einen auf den Stutzen aufgesetzten Schlauch abfließen kann.

Um den auf das Ventil aufgesetzten Adapter in seiner Befestigungslage zu verriegeln, wird ferner vorgeschlagen, dass das napfförmige Gehäuse nahe seinem freien Ende an diametral gegenüber liegenden Stellen zwei durch die Umfangswand verlaufende Schlitze aufweist, in denen zwei parallele Stege einer Verriegelungseinrichtung verschieblich geführt sind. Vorteilhafterweise sind von den Stegen an gegenüber liegenden Stellen Kreisabschnitte weg geschnitten, deren Durchmesser mit dem Innendurchmesser des napfförmigen Gehäuses überein stimmt.

Außerdem wird vorgeschlagen, dass die Enden der parallelen Stege der Verriegelungseinrichtung mit kreisbogenförmigen Querstegen verbunden sind, deren innerer Durchmesser mit dem Außendurchmesser des napfförmigen Gehäuses überein stimmt. Dadurch können die Querstege Anschläge bilden, bei denen die Stege entweder mit den weg geschnittenen Kreisabschnitten an den Schlitzen positioniert sind, so dass der Adapter auf den Endabschnitt eines Ventils aufsetzbar ist, oder die Stege ragen ins Innere des napfförmigen Gehäuses, so dass der Adapter auf dem Ventil verriegelt ist.

Die kreisbogenförmigen Querschnitte können mit Anzeigefahnen für den jeweiligen Verriegelungszustand versehen sein.

Die Erläuterung der Funktion und die Erklärung der Konstruktion folgen im Anschluss als Figurenbeschreibungen.

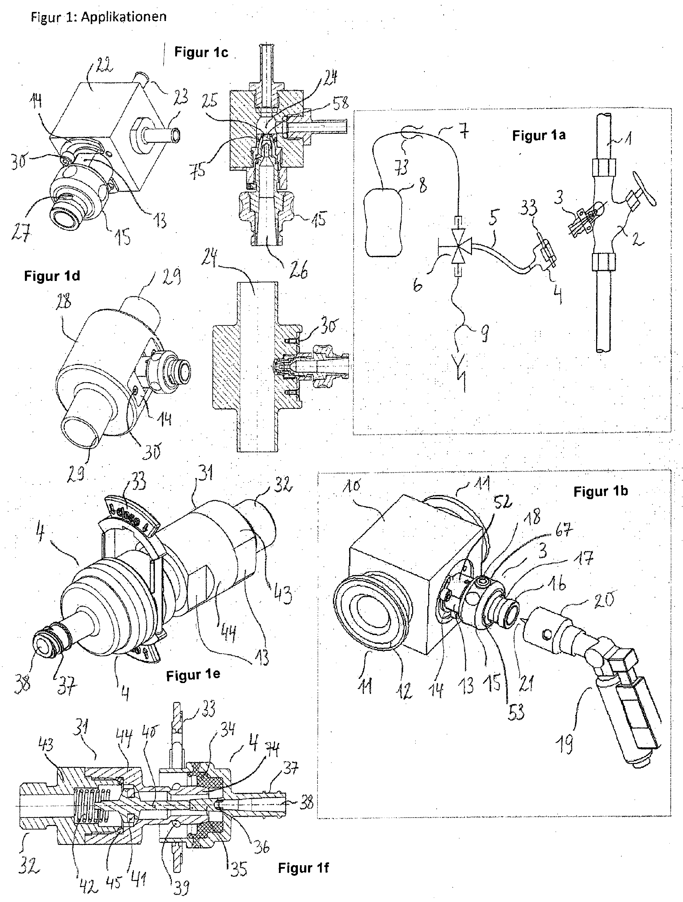

Dabei zeigt Figur 1 a das Prinzip einer kompletten Probeentnahme.

In die flüssigkeitsführende Leitung (1) ist ein Absperrventil (2) mit einer Aufnahmebohrung oberhalb des Absperrschiebers eingebaut. In dieser Aufnahmebohrung kann das Probeentnahmeventil (3) befestigt werden.

Zur Probeentnahme wird der verriegelbare Universaladapter (4) auf das Probeentnahmeventil (3) aufgesteckt und durch Verschieben der Verriegelung (33) dichtend fixiert.

Bei geöffnetem Probeentnahmeventil (3) fließt die Probeflüssigkeit über eine Anschlussleitung (5) zum Dreiwegeventil (6); initial kann über einen Spülanschluss (9) ein Teil der Probe verworfen werden, bevor nach erneutem Betätigen des Dreiwegeventils (6) über einen Behälteranschluss (7) der Probebehälter (8) gefüllt wird. Ist der Probebehälter (8) mit der erforderlichen Menge an Probeflüssigkeit gefüllt, kann eine Klemme (73) geschlossen werden. Das Probeentnahmeventil (3) wird ebenfalls geschlossen und der Universaladapter (4) diskonnektiert. Der verschlossene Probebehälter (8) kann mittels aufgeklebten Identifizierungsdaten in einem Kühlbehälter zum Labor gebracht werden.

Fig. 1b zeigt einen Anschlussblock (10) mit totraumfreien Clampanschlüssen (11), der in eine Rohrleitung oder in eine andere mögliche Anlage lösbar einzubauen ist. Das zweiteilige Probeentnahmeventil (3) ist mit seiner stationären Befestigungseinheit (52) in einen Block (10) eingeschraubt. Damit bei Öffnen- bzw. Schließvorgängen durch die Drehbewegung der Verschlusseinheit (53) der Dichtsitz (75) hin zum Flüssigkeitskanal (24) gesichert bleibt, wird mittels einer Verdrehsicherung (14) die stationäre Befestigungseinheit (52) fixiert. Die Verdrehsicherung (14) ist hufeisenförmig ausgeführt und liegt beidseitig an den Schlüsselflächen (13) der stationären Befestigungseinheit (52) an.

Die drehbare Verschlusseinheit (53) besitzt einen Drehgriff (15), in den umfangseitig die Signaleinheit (67) mit einen Signalknopf (18) eingebracht ist.

Auf die Aufnahmehülse (16) für den Probeadapter kann ein Abflammgerät (19) mit der Zentrierhülse (20) aufgesteckt werden, wobei (21) die Kontur der Flamme zeigt, die direkt in den Probenflüssigkeitskanal (26) zentriert ist.
Fig. 1c zeigt eine Ausführungsform eines Anschlussblocks (22) mit Schlauchanschlüssen (23). Erkennbar ist die Stirnfläche (25) des Probeentnahmeventils (3), die direkt in den Flüssigkeitskanal (24) hineinragt. Ein Dichtkonus (58) verhindert einen Flüssigkeitsaustritt in den Probeflüssigkeitskanal (26).
Fig. 1d zeigt einen Anschlussblock (28) mit Schweißstutzen (29), die in unterschiedlichen Durchmessern ausgeführt und so in pharmazeutische Leitungen eingeschweißt werden können.
Fig. 1e stellt eine mögliche Adaption des Universaladapters (4) an eine Entnahmekupplung (31) dar. Die Entnahmekupplung (31) wird in der Regel mit einem Einschraubgewinde (32) in eine bauseitige Flüssigkeits-Verrohrung eingeschraubt.

Die Entnahmekupplung (31) besteht aus einem Nippelteil (44), welches mit einem Verschlussstück (43) dichtend verschraubt oder verschweißt ist. Im Innern sind ein sternförmiges Verschlussventil (40) mit Dichtung (41) und Andruckfeder (42) angeordnet.

Zur Entnahme von Probeflüssigkeit aus der Kupplung (31) wird der Universaladapter (4) mit der gestuften Dichtung (34) auf das Nippelvorderteil (74) gedrückt. Dabei schiebt ein Öffnungsdorn (35) des Universaladapters (4) das Verschlussventil (40) nach hinten, sodass Probeflüssigkeit über die Flüssigkeitskanäle (36) in den Kanal für Probeflüssigkeit (38) fließen kann. An einem Schlauchanschluss (37) kann eine Anschlussleitung (5) befestigt werden. Bedingt durch die Stufung der Dichtung (34) ist der Adapter (4) an unterschiedliche Durchmesser adaptierbar.

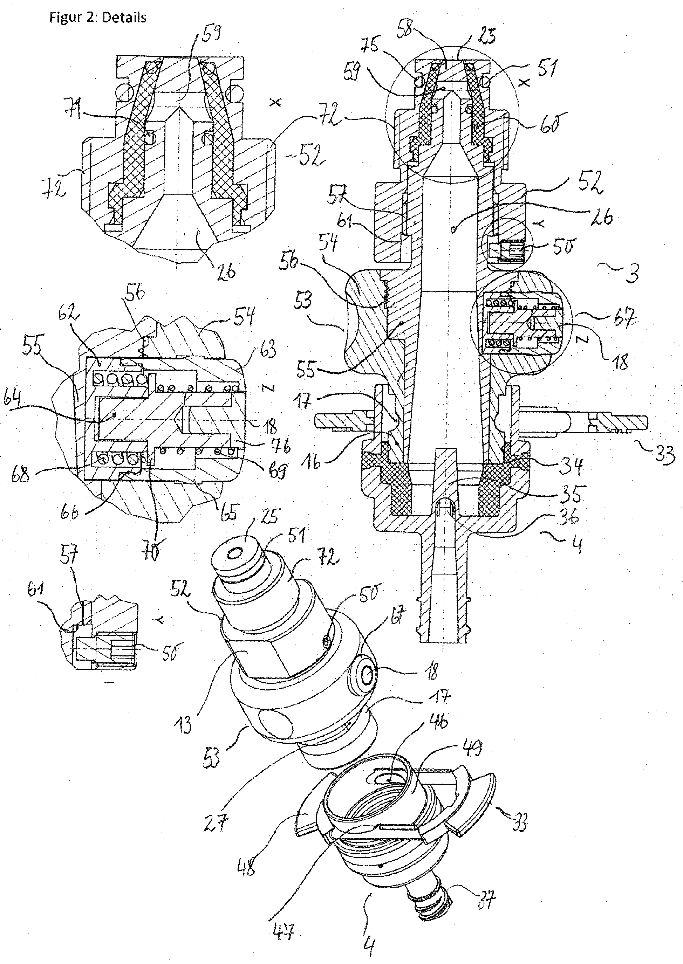

Figur 2 zeigt in vergrößerter, detaillierter Darstellung den Aufbau des Probeentnahmeventils (3), dessen Hauptkomponenten eine stationäre Befestigungseinheit (52) und die drehbare Verschlusseinheit (53) sind.

Die stationäre Befestigungseinheit (52) ist mit einem Gewinde (72) beispielsweise in eine der vorgenannten Applikationen hineinzuschrauben. Möglich sind anstelle eines Gewindes auch Rast - oder Steckanschlüsse.

Das der flüssigkeitsführenden Leitung (1; 24) zugewandte Ende (25) weist einen Dichtkonus (58) zum Probeflüssigkeitskanal (26) auf. Zur weiteren Verbesserung der Dichteigenschaften und als möglicher thermischer Isolator kann ein Probekern-Dichtsatz (60) vorzugsweise aus Teflon oder einem ähnlichen Material eingesetzt werden. Die Dichtung (75) zeigt eine Dichtungsmöglichkeit der stationären Befestigungseinheit (52) zum Einbauort hin. Mit dem Verschlussgewinde (57) wird die Verschlusseinheit (53) bis maximal zum Anschlag (61) bzw. Herausdrehsicherung (50) geöffnet.

Die Verschlusseinheit (53) selbst weist einen metallischen Probekern (55) auf, der voll umfänglich von einem Griffstück mit Isolation (54) umschlossen ist.

Durch Drehbewegung des Griffstücks (54/15) bewegt sich der Probekern (55) mittels des Verschlussgewindes (57) und gibt die Konusdichtung (58) frei, sodass Probeflüssigkeit über den T-förmig angeordneten Probeeinlaufkanal (59) zum Probenflüssigkeitskanal (26) gelangt.

Die Verbindung des metallischen Probekerns (55) mit dem isolierenden, vorzugsweise aus Kunststoff hergestellten Griffstück (54) wird formschlüssig durch eine Aufnahme (56), die als Rast -oder Rändelaufnahme ausgeführt sein kann, hergestellt.

Die Signaleinheit (67) ist mit einem Wärmeleittopf (62) formschlüssig in dem Kern (55) eingebracht und mittels einer Signaleinheitverrastung (65) in das Griffstück (54) eingesetzt und dadurch mit der Verschlusseinheit (53) verbunden.

Die Signaleinheit (67) ist eine zweiteilige verbundene Einheit, deren unterer Wärmeleittopf mittels Verrastung (66) mit der Signalknopfhülse (63) verbunden ist. Innerhalb der Signaleinheit (67) ist in den Wärmeleittopf (62) eine Formgedächtnisfeder (68) eingesetzt, die im Arbeitsfall eine Signalknopfführung (64) so bewegt, dass der Signalknopf (18) deutlich über den Rand der Signalknopfhülse (63) herausragt. In der Ruhephase wird Signalknopfführung (64) durch Rückstellfeder (69) zurück gedrückt.

Der Signalknopf (18) und die Signalknopfführung (64) sind miteinander formschlüssig verbunden und können durch Dimensionierung des Anschlags (70) bzw. Tiefe und Farbe des Bundes (76) zum einen ein eindeutiges Arbeitssignal liefern und zum anderen auf unterschiedliche Temperaturen eingestellt werden.

Bei nicht dargestelltem Eintritt der Flamme (21) in Probenflüssigkeitskanal (26) wird der Probekern (55) erwärmt. Der Wärmeübertrag erhöht ebenfalls die Temperatur des Wärmeleittopfes (62), in dem die Formgedächtnisfeder (68) angeordnet ist. Mit zunehmender Temperatur drückt die Formgedächtnisfeder (68) gegen den Anschlag (70) die Signalknopfführung (64) so nach außen, dass ein deutlicher Übertritt des Signalknopfes (18) über der Hülse (63) entsteht.

Vorzugsweise wird die Signalknopfführung (64) in einer anderen Farbe als der Signalknopf (18) dargestellt, sodass nach deutlichem Übertritt über die Hülse (63) auch ein Farbwechsel erkennbar ist.

Bei Erkaltung von Formgedächtnisfeder (68) drückt eine Rückstellfeder (69) den Signalknopf gegen den Bund (7) in die Ruheposition zurück.

Nach dem Abflammvorgang und Registrierung der Signalknopfanzeige kann der Universaladapter (4) auf Aufnahme (16) gesteckt und mittels Verriegelung (33) an der Nut (17) verriegelt werden. Dazu ist die Verriegelung (33) in "Auf"-Position gestellt, sodass die zylindrische Aufsteckposition (46) ein Aufschieben des Adapters (4) ermöglicht. Die Position der Verriegelung ist auch an der Aufschrift an den Verriegelungsfahnen (48) ersichtlich.

Durch Herunterdrücken von Verriegelung (33) entsteht eine seitliche Führung und Fixierung in der Nut (17). Im aufgesteckten Zustand kann an dem Anschluss (37) die Leitung (5) konnektiert und die Verschlusseinheit (53) so geöffnet werden, dass die Probeflüssigkeit entnommen werden kann.

Es wird betont, dass die Erfindung nicht auf die beschriebenen und dargestellten Ausführungsformen beschränkt ist, Vielmehr sind alle offenbarten Merkmale auf jede sinnvolle Weise einzeln miteinander kombinierbar.

**Legende**

| | |
|---|---|
| 1. | Flüssigkeitsführende Leitung |
| 2. | Ventil mit Probenaufnahme |
| 3. | Probeentnahmeventil |
| 4. | Probeadapter |
| 5. | Anschlussleitung |
| 6. | Dreiwegeventil |
| 7. | Behälteranschluss mit Schlauchklemme |
| 8. | Probebehälter |
| 9. | Spülanschluss |
| 10. | Anschlussblock mit Clampanschlüsse |
| 11. | Totraumfreie Clampanschlüsse |
| 12. | O-Ringaufnahme |
| 13. | Schlüsselfläche |
| 14. | Verdrehsicherung |
| 15. | Drehgriff |
| 16. | Aufnahmehülse für Probeadapter |
| 17. | Verriegelungsnut |
| 18. | Signalknopf |
| 19. | Abflammgerät |
| 20. | Zentrierhülse |
| 21. | Flamme |
| 22. | Anschlussblock mit Schlauchanschlüssen |
| 23. | Schlauchtüllen |
| 24. | Flüssigkeitskanal |
| 25. | Stirnfläche Probeentnahmeventil |
| 26. | Probenflüssigkeitskanal |
| 27. | Hilfsschlüsselfläche |
| 28. | Anschlussblock mit Anschweißstutzen |
| 29. | Anschweißstutzen |
| 30. | Befestigungsschrauben für Verdrehsicherung |
| 31. | Entnahmekupplung mit Verschlussventil |
| 32. | Einschraubgewinde |
| 33. | Verriegelung |
| 34. | Gestufte Dichtung |
| 35. | Öffnungsdorn |
| 36. | Flüssigkeitskanäle |
| 37. | Schlauchanschluss Probeadapter |
| 38. | Kanal Probeflüssigkeit |
| 39. | Verriegelungsnut Entnahmekupplung |
| 40. | Sternförmiges Verschlussventil |
| 41. | Dichtung Verschlussventil |
| 42. | Andruckfeder |
| 43. | Kupplungs- Verschlussstück |
| 44. | Kupplungs- Nippelteil |
| 45. | Dichtung Verschluss - u. Nippelteil |
| 46. | Verriegelung - Aufsteckposition |
| 47. | Verriegelungsführung |
| 48. | Verriegelungsfahne |
| 49. | Adapterhülse |
| 50. | Herausdrehsicherung/ Anschlag |
| 51. | Dichtungssitz |
| 52. | Stationäre Befestigungseinheit |
| 53. | Verschlusseinheit |
| 54. | Griffstück mit Isolation |
| 55. | Probekern |
| 56. | Isolationsaufnahme |
| 57. | Verschlussgewinde |
| 58. | Dichtkonus |
| 59. | Probeeinlaufkanal |
| 60. | Probekern-Dichtsitz |
| 61. | Ausdrehanschlag |
| 62. | Wärmeleittopf |
| 63. | Signalknopfhülse |
| 64. | Signalknopfführung |
| 65. | Signaleinheitverrrastung |
| 66. | Wärmeleittopfverrastung |
| 67. | Signaleinheit |
| 68. | Formgedächtnisfeder |
| 69. | Rückstellfeder |
| 70. | Federanschlag |
| 71. | Kerndichtung |
| 72. | Gewinde oder Steckzylinder |
| 73. | Klemme |
| 74. | Nippelvorderteil |
| 75. | Dichtsitz |
| 76. | Bund Signalknopf |
| 77. | |

## Patentansprüche

1. Probeentnahmeventil, mit einem Ventilkörper (55), der in einem Ventilgehäuse (52) zwischen einer Schließstellung und einer Öffnungsstellung des Probeentnahmeventils bewegbar ist, wobei der Ventilkörper (55) einen Probenflüssigkeitskanal (26) aufweist und aus Metall besteht,
**dadurch gekennzeichnet,**
**dass** eine Signaleinheit mit einem im Wesentlichen topfförmigen Gehäuseteil (62) aus Metall mit dem Ventilkörper (55) in Berührung steht,
**dass** die Signaleinheit einen Signalknopf (18) enthält, der von einer Formgedächtnisfedereinrichtung (68) bei deren Erwärmung aus einem Gehäuse (62, 63) vorschiebbar ist,
und **dass** in dem Gehäuse (62, 63) außerdem eine Rückstellfeder (69) angeordnet ist, die eine kleinere Federkraft hat als die Formgedächtnisfeder (68) und den Signalknopf (18) in die zurückgezogene Ausgangsposition vorspannt.

2. Probeentnahmeventil nach Anspruch 1,
**dadurch gekennzeichnet,**
**dass** außen auf dem Ventilkörper (55) ein Griffstück (54) aus einem wärmeisolierenden Material sitzt.

3. Probeentnahmeventil nach Anspruch 1 oder 2,
**dadurch gekennzeichnet,**
**dass** das Gehäuse aus dem topfförmigen Gehäuseteil (62) und einem damit verbundenen Gehäuseteil (63) aus Kunststoff besteht und in das Griffstück (54) eingelassen ist.

4. Probeentnahmeventil nach den Ansprüchen 1 bis 3,
**dadurch gekennzeichnet,**
**dass** in dem Gehäuse (62, 63) ein mit einem Bund (70) versehener Führungskörper (64) verschieblich angeordnet ist, an dessen freiem Ende der Signalknopf (18) befestigt ist.

5. Probeentnahmeventil nach den Ansprüchen 1 bis 4,
**dadurch gekennzeichnet,**
**dass** die Formgedächtnisfeder (68) sich am Boden des topfförmigen Gehäuses (62) und an dem Bund (70) abstützt.

6. Probeentnahmeventil nach den Ansprüchen 4 oder 5 **dadurch gekennzeichnet,**
**dass** der Signalknopf (18) eine andere Farbe als der Führungskörper (64) aufweist und dass bei entsprechender Erwärmung des Ventilkörpers (55) der Signalknopf (18) und ein Endabschnitt des Führungskörpers (64) über das Griffstück (54) hinaus vorschiebbar sind.

7. Probeentnahmeventil nach den Ansprüchen 1 bis 6,
**dadurch gekennzeichnet,**
**dass** der Ventilkörper (65) am stirnseitigen Ende einen Dichtkonus (58) aufweist, mit dem die Proben-Eintrittsöffnung des Ventilgehäuses (52) dicht verschließbar ist.

8. Probeentnahmeventil nach den Ansprüchen 1 bis 7,
**dadurch gekennzeichnet,**
**dass** der Ventilkörper (55) in das Ventilgehäuse (52) eingeschraubt ist.

9. Probeentnahmeventil nach den Ansprüchen 1 bis 8,
**dadurch gekennzeichnet,**
**dass** auf dem freien Endabschnitt des Griffstücks (54) ein Probeadapter (4) befestigbar ist, der einen Schlauchanschluss (37) aufweist.

## Claims

1. A sampling valve with a valve body (55), which is movable within a valve housing (52) between a closed position and an open position of the sampling valve, wherein the valve body (55) includes a sample liquid passage (26) and consists of metal, **characterised in that** a signal unit with a substantially cup-shaped housing portion (62) of metal is in contact with the valve body (55), that the signal unit includes a signal button (18), which may be advanced out of a housing (62, 63) by a shape memory spring device (68), when the latter is heated, and that a return spring (69) is also arranged in the housing (62, 63), which has a smaller spring force than the shape memory spring (68) and biases the signal button (18) into the retracted starting position.

2. A sampling valve as claimed in Claim 1, **characterised in that** a handle member (54) of a heat-insulating material is situated externally on the valve body (55).

3. A sampling valve as claimed in Claim 1 or 2, **characterised in that** the housing consisting of the cup-shaped housing portion (62) and a housing portion (63) connected thereto consists of plastic material and is set into the handle member (54).

4. A sampling valve as claimed in Claims 1 to 3, **characterised in that** a guide body (64) provided with a flange (70) is movably arranged in the housing (62, 63), secured to the free end of which is the signal button (18).

5. A sampling valve as claimed in Claims 1 to 4, **characterised in that** the shape memory spring (68) bears against the base of the cup-shaped housing (62) and against the flange (70).

6. A sampling valve as claimed in Claims 4 or 5, **characterised in that** the signal button (18) has a different colour than the guide body (64) and that when the valve body (55) is appropriately heated the signal button (18) and an end section of the guide body (64) may be advanced beyond the handle member (54).

7. A sampling valve as claimed in Claims 1 to 6, **characterised in that** the valve body (65) includes a sealing cone (58) at the end, with which the sample inlet opening of the valve housing (52) may be closed in a sealed manner.

8. A sampling valve as claimed in Claims 1 to 7, **characterised in that** the valve body (55) is screwed into the valve housing (52).

9. A sampling valve as claimed in Claims 1 to 8, **characterised in that** a sample adapter (4), which includes a hose connection (37), may be secured to the free end section of the handle member (54).

## Revendications

1. Soupape de prélèvement d'échantillon avec un corps de soupape (55) qui est mobile dans un boîtier de soupape (52) entre une position de fermeture et une position d'ouverture de la soupape de prélèvement d'échantillon, dans laquelle le corps de soupape (55) présente un canal de liquide d'échantillon (26) et se compose de métal,
**caractérisée en ce**
**qu'**une unité de signal avec une partie de boîtier (62) sensiblement en forme de pot en métal est en contact avec le corps de soupape (55),
**que** l'unité de signal contient un bouton de signal (18) qui peut être avancé d'un boîtier (62, 63) par un dispositif de ressort à mémoire de forme (68) lors de son réchauffement,
et **que** dans le boîtier (62, 63), un ressort de rappel (69) est en outre agencé, lequel possède une force de ressort plus petite que le ressort à mémoire de forme (68) et précontraint le bouton de signal (18) dans la position de départ retirée.

2. Soupape de prélèvement d'échantillon selon la revendication 1,
**caractérisée en ce**
**qu'**une pièce de préhension (54) en un matériau thermoisolant loge à l'extérieur sur le corps de soupape (55).

3. Soupape de prélèvement d'échantillon selon la revendication 1 ou 2,
**caractérisée en ce**
**que** le boîtier se compose d'une partie de boîtier (62) en forme de pot et une partie de boîtier (63) reliée à celle-ci en matière plastique et est encastré dans la pièce de préhension (54).

4. Soupape de prélèvement d'échantillon selon les revendications 1 à 3,
**caractérisée en ce**
**que** dans le boîtier (62, 63), un corps de guidage (64) pourvu d'un collet (70) est agencé de manière mobile, sur l'extrémité libre duquel le bouton de signal (18) est fixé.

5. Soupape de prélèvement d'échantillon selon les revendications 1 à 4,
**caractérisée en ce**
**que** le ressort à mémoire de forme (68) s'appuie sur le fond du boîtier (62) en forme de pot et sur le collet (70).

6. Soupape de prélèvement d'échantillon selon les revendications 4 ou 5,
**caractérisée en ce**
**que** le bouton de signal (18) présente une autre couleur que le corps de guidage (64) et en ce que lors du réchauffement correspondant du corps de soupape (55), le bouton de signal (18) et une section d'extrémité du corps de guidage (64) peuvent être avancés au-delà de la pièce de préhension (54).

7. Soupape de prélèvement d'échantillon selon les revendications 1 à 6,
**caractérisée en ce**
**que** le corps de soupape (65) présente sur l'extrémité côté avant un cône étanche (58), avec lequel l'ouverture d'entrée d'échantillon du boîtier de soupape (52) peut être fermée de manière étanche.

8. Soupape de prélèvement d'échantillon selon les revendications 1 à 7,
**caractérisée en ce**
**que** le corps de soupape (55) est vissé dans le boîtier de soupape (52).

9. Soupape de prélèvement d'échantillon selon les revendications 1 à 8,
**caractérisée en ce**
**qu'**un adaptateur d'échantillon (4) peut être fixé sur la section d'extrémité libre de la pièce de préhension (54), lequel présente un raccord de tuyau (37).
